# EUROPÄISCHE PATENTSCHRIFT

(11) **EP 0 761 650 B1**
(45) Veröffentlichungstag und Bekanntmachung des Hinweises auf die Patenterteilung: **31.10.2001**
(21) Anmeldenummer: 96112489.8
(22) Anmeldetag: 02.08.1996
(51) Int. Cl.: C07D 207/12, A61K 31/40

(54) **Thermostabile und lagerfähige Kristallmodifikation von N-Methyl-N-((1S)-1-phenyl-2-((3S)-3-hydroxypyrrolidin-1-yl)-ethyl)-2,2-diphenyl-acetamid und Verfahren zu dessen Herstellung**
Thermally stable and storable crystalline modification of N-methyl-N-((1S)-1-phenyl-2-((3S)-3-hydroxypyrrolidin-1-yl)-ethyl)-2,2-diphenyl-acetamide and process for its preparation
Modification cristalline thermostable et supportant le stockage du N-méthyle-N-((1S)-1-phényle-2-((3S)-3-hydroxypyrrolidin-1-yl)-éthyle)-2,2-diphényle-acétamide et procédé pour sa préparation

(30) Priorität: 26.08.1995 DE 19531464
(43) Veröffentlichungstag der Anmeldung: 12.03.1997
(73) Patentinhaber: MERCK PATENT GmbH, 64293 Darmstadt (DE)
(72) Erfinder: Stein, Inge, Dr., 63110 Rodgau (DE); Beeres, Holger, 64560 Crumstadt (DE); Beschmann, Klaus, Dr., 64354 Reinheim (DE); Neuenfeld, Steffen, Dr., 64409 Messel (DE); Barber, Andrew, Dr., 64331 Weiterstadt (DE)

(56) Entgegenhaltungen:
- EP-A- 0 569 802
- BIOORGANIC & MEDICINAL CHEMISTRY LETTERS, Bd. 4, Nr. 5, 1994, Seiten 677-682, XP000602924 GOTTSCHLICH R. ET AL.: "EMD 61753 as a favorable representative of structurally novel arylacetamido-type .kappa. opiate receptor agonists"
- CHIRALITY , Bd. 6, Nr. 8, 1994, Seiten 685-689, XP000602923 GOTTSCHLICH R. ET AL.: ".Kappa.-opioid activity of the four stereoisomers of the peripherally selective .kappa.-agonists, EMD 60 400 and EMD 61 753"
- JOURNAL OF PHARMACEUTICAL SCIENCES, Bd. 58, Nr. 8, August 1969, Seiten 911-929, XP002020518 HALEBLIAN J. & MC CRONE W.: "Pharmaceutical applications of polymorphism"
- MANUFACTURING CHEMIST & AEROSOL NEWS, Bd. 44, Nr. 12, Dezember 1973, Seite 37,40 XP002020519 PEARSON J.T. & VARNEY G.: "The influence of crystal structure on drug formulation"
- JOURNAL OF PHARMACEUTICAL SCIENCES, Bd. 64, Nr. 8, August 1975, Seiten 1269-1288, XP002020520 HALEBLIAN J.K.: "Characterization of habits and crystalline modification of solids and their pharmaceutical applications"

## Beschreibung

Die Erfindung betrifft eine neue thermostabile Form von N-Methyl-N-[(1 S-)-1-phenyl-2-((3S)-3-hydroxypyrrolidin1-yl-)-ethyl]-2,2-diphenyl-acetamid und ein Verfahren zur Herstellung und Isolierung dieser Verbindung in dieser neuen Form sowie die Verwendung zur Herstellung von Arzneimitteln, die diese Verbindung und/ oder eines ihrer physiologisch unbedenklichen Salze enthalten.

Verbindungen mit dieser Strukturformel sowie auch die oben genannte Verbindung und geeignete Verfahren zu deren Herstellung sind in der Offenlegungsschrift DE 42 15 213 A1 beschrieben.

Die Zeitschriftenartikel von Gottschlich R. et al, Bioorg. & Med. Chem. Lett. 1994, 4(5), 677-682 und Chirality, 1994, 6(8), 685-689, beschreiben die Verbindung N-Methyl-N-[(1S-)-1-phenyl-2-((3S)-3-hydroxypyrrolidin1-yl-)-ethyl]-2,2-diphenyl-acetamid (EMD 61753), hergestellt nach der Lehre der DE 42 15 213 A1, als strukturell neuen Kappa-Opiat-Rezeptor-Agonisten.

Die Veröffentlichungen von Haleblian J. & McCrone W, Journal of Pharmaceutical Sciences, 1969, 58(8), 911-929, Pearson J.T. & Varney G, Manufacturing Chemist & Aerosol News, 1973, 44(12), S. 37 und 40 sowie Haleblian J.K., Journal of Pharmaceutical Sciences, 1975, 64(8), 1269-1288 sind Übersichtsartikel, die die Polymorphie von pharmazeutischen Wirkstoffen und die Bedeutung der polymorphen Formen für eine pharmazeutische Formulierung eines Wirkstoffs erläutern.

Es wurde gefunden, daß die bereits aus der Patentanmeldung DE 42 15 213 A1 bekannte Verbindung N-Methyl-N-[(1S-)-1-phenyl-2-((3S)-3-hydroxypyrrolidin1-yl-)-ethyl]-2,2-diphenyl-acetamid, eine pharmazeutisch besonders wirksame Verbindung ist, die sich als Arzneimittel zur Behandlung entzündlicher Darmerkrankungen in ganz besonderer Weise eignet. Insbesondere ist diese Verbindung bei dieser Indikation einsetzbar und wirksam, da sie gleichzeitig die mit dieser Erkrankung verbundenen Schmerzen lindert und im akuten Fall eines durch die entzündliche Darmerkrankung drohenden bzw. hervorgerufenen Darmverschlußes die Motorik des Darms wieder normalisiert oder wieder in Gang setzt, ohne spürbare Nebenwirkungen hervorzurufen.

Versuche zur Herstellung von nach dem aus DE 42 15 213 A1 bekannten Verfahren haben gezeigt, daß diese Verbindung in verschiedenen Formen erhalten wird.

Aufgabe der Erfindung war es daher, N-Methyl-N-[(1S-)-1-phenyl-2-((3S)-3-hydroxypyrrolidin1-yl-)-ethyl]-2,2-diphenyl-acetamid in thermostabiler Form zur Verfügung zu stellen und ein Verfahren zur Herstellung dieser Verbindung bereit zu stellen, wodurch ein thermostabiles Produkt erhalten wird, das lagerstabil und zur Herstellung von Arzneimittelformulierungen geeignet ist.

Gegenstand der Erfindung ist somit thermodynamisch stabiles, lagerfähiges N-Methyl-N-[(1S-)-1-phenyl-2-((3S)-3-hydroxypyrrolidin1-yl-)-ethyl]-2,2-diphenyl-acetamid mit einem Schmelzpunkt von 220-225°C sowie dessen Verwendung als Arzneimittel zur Behandlung von entzündlichen Darmerkrankungen auch pharmazeutische Zubereitungen, die diese Verbindung als Bestandteil enthalten und daher zur wirksamen Behandlung entzündlicher Darmerkrankungen und der damit verbundenen Krankheitssymptome, sowie zur Behandlung von starken Schmerzen, insbesondere von Schmerzüberempfindlichkeit, einsetzbar sind.

Gegenstand der Erfindung ist ebenfalls die Verwendung dieser Verbindung als Arzneimittel zur Behandlung von Schmerzen und Schmerzüberempfindlichkeit auftretend bei Rückenleiden, Brandverletzungen, Sonnenbrand und rheumatischen Erkrankungen, sowie dabei auftretenden entzündlichen Reaktionen. Gegenstand der Erfindung ist auch die Verwendung dieses Arzneimittels zur Behandlung von postoperativen Schmerzen, Schmerzüberempfindlichkeitsreaktionen, sowie des häufig nach Abdominaloperationen auftretenden Ileus. Ein weiterer Gegenstand der Erfindung ist die Verwendung der entsprechenden Verbindung in Arzneimittelformulierungen zur Behandlung von Neurodermitis.

Darüber hinaus ist ein Verfahren zur Herstellung von thermodynamisch stabilem, lagerfähigem N-Methyl-N-[(1S-)-1-phenyl-2-((3S)-3-hydroxypyrro-lidin1-yl-)-ethyl]-2,2-diphenyl-acetamid mit einem Schmelzpunkt von 220-225°C Gegenstand dieser Erfindung.

Die erfindungsgemäße Verbindung und ihre physiologisch unbedenklichen Salze zeigen besonders gute analgetische Wirkungen. In diesem Zusammenhang antagonisieren sie insbesondere entzündungsbedingte Hyperalgesien, sind aber auch wirksam in der Bekämpfung des eigentlichen Entzündungsgeschehens, so daß sie ein breites Wirkungsspektrum aufweisen.

Versuche haben gezeigt, daß die erfindungsgemäße Verbindung im "Writhing Test" an Mäusen oder Ratten wirken(Methode vgl. Siegmund et. al., Proc. Soc. Exp. Biol. 95, (1957), 729-731). Die analgetische Wirkung als solche läßt sich ferner im "Tail-Flick-Test" an Mäusen oder Ratten nachweisen (Methodik vgl. d'Amour and Smith, J. Pharmacol. Exp. Ther. 72, (1941), 74-79), ferner im "Hot plate test" (vgl. Schmauss und Yaksh, J. Pharmacol. Exp. Ther. 228, (1984), 1-12 und die dort zitierte Literatur). Besonders starke Wirkungen sind an Ratten im Modell der Carrageenin-induzierten Hyperalgesie (vgl. Bartoszyk und Wild, Neuroscience Letters 101 (1989) 95) zu beobachten. Dabei zeigt diese Verbindung keine oder nur geringe Neigung zu physischer Abhängigkeit.

Außerdem wurden durch entsprechende nach geläufigen Methoden durchgeführte Versuche ausgeprägte antiinflammatorische, diuretische, antikonvulsive, neuroprotektive Wirkungen nachgewiesen. Die Verbindung zeigt eine hohe Affinität in bezug auf das Bindungsverhalten an kappa-Rezeptoren.

Die erfindungsgemäße Verbindung ist im Gegensatz zu anderen Verbindungen mit ähnlichem Wirkungsspektrum besonders geeignet für die Verwendung in pharmazeutischen Zubereitungen zur Behandlung entzündlicher Darmerkrankungen, da sie neben der analgetischen und antiinflammatorischen Wirkung geeignet ist, durch die Erkrankung hervorgerufene Störungen der Darmmotorik zu normalisieren. Insbesondere ist sie geeignet, die Darmbewegungen wieder in Gang zu bringen, wenn durch die entzündliche Darmerkrankung ein Darmverschluß droht oder bereits eingetreten ist. Auch kann diese Wirkung zur Behandlung eines postoperativen lleus und der damit verbundenen Schmerzen eingesetzt werden.

Die erfindungsgemäße Verbindung hat sich aufgrund der oben beschriebenen pharmakologischen Wirksamkeit als besonders geeignet erwiesen in der Behandlung von Verbrennungen, und zwar sowohl von Verbrennungen durch Hitze- oder Flammeneinwirkung als auch von starken Sonnenbränden. Insbesondere lassen sich bei diesen Indikationen durch die Verabreichung von geeigneten pharmazeutischen Zubereitungen, die den erfindungsgemäßen Wirkstoff enthalten, neben den eigentlichen Schmerzen und Schmerzüberempfindlichkeitsreaktionen entzündliche Vorgänge mit beeinflussen. Auch läßt sich der bei schwersten Verbrennungen auftretende reflektorische lleus verhindern, bzw. behandeln.

In diesem Zusammenhang wurden auch Anzeichen gefunden, die auf eine vorteilhafte Wirkung in der Behandlung von Sonnenallergien hinweisen, zumal unter Einfluß der erfindungsgemäßen Verbindung allergische Hautreaktionen schnell abklingen und der damit verbundene Juckreiz schnell nachläßt. Entsprechende positive Ergebnisse wurden auch in der Behandlung von Neurodermitis gefunden. Insbesondere läßt bei dieser Erkrankung unter Einwirkung des oben genannten Wirkstoffs der Juckreiz der Haut nach und durch die Erkrankung auftretende entzündliche Reaktionen werden günstig beeinflußt.

Weiterhin hat sich dieser Wirkstoff als besonders wirksam in der Behandlung von rheumatischen Erkrankungen und von Rückenleiden erwiesen. Besonders vorteilhaft ist in diesem Zusammenhang, daß er sowohl gegen die damit verbundenen Schmerzen wirksam ist als auch die bei rheumatischen Erkrankungen auftretenden entzündlichen Vorgänge positiv beeinflußt und so zu einer Verbesserung des Allgemeinbefindens des Patienten beiträgt. Hierbei hat sich vorteilhafterweise gezeigt, daß eine normale Motorik des Magen-Darmtraktes nicht negativ beeinflußt wird.

In allen hier beschriebenen Indikationsgebieten hat sich die Verwendung von N-Methyl-N-[(1S-)-1-phenyl-2-((3S)-3-hydroxypyrrolidin-1-yl-)-ethyl]-2,2-diphenyl-acetamid-hydrochlorid als Arzneimittel in den verschiedensten Zubereitungsformen als besonders wirksam herausgestellt.

Chemische und physikalische Untersuchungen zur Charakterisierung dieser erfindungsgemäßen Verbindung haben gezeigt, daß sie je nach Herstellung und Lagerung in verschiedenen Formen erhalten wird.

Im einzelnen wurden bisher vier verschiedene Erscheinungsformen gefunden, die sich erheblich voneinander unterscheiden. Typ I ist ein Solvat. Diese Form wandelt sich leicht in einen anderen Kristalltyp um, der im folgenden Text als Typ II bezeichnet wird. Bei langsamem Erhitzen von Typ I findet eine Solvensabgabe statt. Anschließend entsprechen die Daten der Röntgendiffraktometrie-, IR-Analyse und der Schmelzpunkt denen des Typs II.

Die nach dem bisher bekannten und in der früheren Anmeldung DE 4215 213 A1 beschriebenen Verfahren hergestellte Verbindung wird als Typ II erhalten und besitzt einen Schmelzbereich von 196-200 °C (siehe auch (Bioorganic & Medicinal Chemistry Letters 4 (5), 679 (1994)) und weist eine Schmelzwärme von 100 J/g auf.

Eine als Typ III bezeichnete Form wird erhalten, wenn Typ II unter extremen Bedingungen bei einer Luftfeuchtigkeit von etwa 73 bis 95 % gelagert wird.

Eine im folgenden als Typ IV charakterisierte Modifikation besitzt gegenüber der des Typs II einen Schmelzbereich von etwa 220-226 °C und eine Schmelzwärme von etwa 120-128 J/g.

Der höhere Schmelzpunkt und die höhere Schmelzwärme der erfindungsgemäßen Verbindung zeigen, daß die thermodynamisch stabile Kristallmodifikation vorliegt, wobei die beiden erhaltenen Kristallmodifikationen II und IV monotrop zueinander sind.

Dieser Sachverhalt kann durch weitere Beobachtungen bestätigt werden. Beim sehr schnellen Abkühlen einer aus thermodynamisch nicht stabilen Kristallmodifikation (Fp. 196- 200 °C) erhaltenen Schmelze von 210 °C auf -78 °C und 5-stündiger Lagerung bei -78 °C kristallisiert langsam die thermostabile Verbindung (221-226 °C) aus.

Es unterscheiden sich bei diesen beiden Typen nicht nur deren Schmelzverhalten. Sie unterscheiden sich auch in ihren Lagerstabilitäten. Es wurde nach Lagerung des Typs II im Trockenschrank bei 170 °C eine teilweise Umwandlung in Typ IV nachgewiesen. Während der DSC-Bestimmung (DSC = Differential Scanning Calorimetry) der Kristallmodifikation des Typs II kristallisiert bei langsamem Erhitzen oberhalb des Schmelzpunkts bei etwa 200 °C der Kristalltyp IV aus, der bei etwa 220 - 226 °C schmilzt. Kristalltyp IV wird auch nachgewiesen, wenn eine Schmelze vom Typ II (knapp oberhalb 200 °C) schlagartig abgekühlt wird und für eine Zeit von 12 bis 16 Stunden bei Raumtemperatur gelagert wird. Während sich Typ II daher metastabil erwiesen hat, ist Typ IV die thermodynamisch stabile Modifikation. Durch gezielte Kristallisationsversuche wurde gefunden, daß durch Zugabe von Impfkristallen des Typs IV nur die Kristallmodifikation IV erhalten wird. Im Gegensatz dazu wurde jedoch durch Animpfen mit Impfkristallen des Typs II vor allem die Kristallmodifikation IV erhalten. Außerdem ist die Löslichkeit des Kristalltyps IV in wäßriger Lösung niedriger als die des Typs II. Sie beträgt etwa 45 bis 70 % der Löslichkeit des Typs II.

Nach siebenmonatiger Lagerung der nach dem in der früheren Anmeldung DE 4215213 A1 beschriebenen Verfahren hergestellten Verbindung bei Raumtemperatur konnte eine beginnende Umwandlung zur thermostabilen Kristallmodifikation IV festgestellt werden. Das heißt, die nach dem in dieser Erfindung beschriebenen Verfahren hergestellte Verbindung ist im Gegensatz zur anderen Kristallmodifikation lagerstabil.

In Gegenwart von Lösungsmitteln (z. B. Wasser) kann die hergestellte Verbindung Solvate bilden.

Bei Verwendung der erfindungsgemäßen Verbindung mit der Kristallmodifikation des Typs II in Arzneimittelformulierungen kann es bei längerer Lagerung zu einer Umwandlung der Kristallmodifikationen kommen.

Bisherige Untersuchungen der Arzneimittelwirksamkeit haben sich zwar bislang als relativ unabhängig von der Kristallmodifikation gezeigt, was jedoch Unterschiede durch eine möglicherweise geänderte Bioverfügbarkeit nicht ausschließt. Für die Verwendung in festen Arzneimittelformulierungen muß daher darauf geachtet werden, daß die stabile Kristallmodifikation des Typs IV eingesetzt wird, bzw. darauf, daß bei Verwendung des Typs II Additive eingesetzt werden, durch die eine langfristige Stabilisierung dieses Kristalltyps erfolgt.

Überraschenderweise zeigte sich nun, daß nicht nur die Art und Weise der Aufarbeitung des nach der eigentlichen Reaktion erhaltenen Rohprodukts einen Einfluß auf die Modifikation der Kristalle hat, sondern daß dafür bereits die Bedingungen unter denen die Edukte miteinander reagieren von Bedeutung sind. Es wurde gefunden, daß hierbei insbesondere die Reaktionstemperaturen und die Lösungsmittelverhältnisse eine Rolle spielen.

Im einzelnen hat sich gezeigt, daß der Kristallisationstyp IV erhalten wird, wenn die Umsetzung der Edukte 1-[(1S)-3-Hydroxypyrrolidin-1-yl]-(2S)-2-methylamino-2-phenyl-ethan und Diphenylacetylchlorid bei niedrigen Temperaturen , insbesondere bei -5 bis 10 °C, bevorzugt bei 0 bis 8 °C erfolgt.

Es hat sich weiterhin für diesen Zweck als vorteilhaft erwiesen , daß das molare Verhältnis der Edukte 1-[(1S)-3-Hydroxypyrrolidin-1-yl]-(2S)-2-methylamino-2-phenyl-ethan und Diphenylacetylchlorid zueinander 1:0,75 bis 1:1,65, vorzugsweise 1:1,1 bis 1:1,3 beträgt.

Das molare Verhältnis der Ausgangsverbindungen 1-[(1S)-3-Hydroxypymolidin-1-yl]-(2S)-2-methylamino-2-phenyl-ethan und Diphenylacetylchlorid zu dem verwendeten Lösungsmittel sollte dabei so gewählt werden, daß die Edukte in Lösung sind, das Lösungsmittel jedoch möglichst nur in geringem Überschuß vorliegt. Wird Tetrahydrofuran als Lösungsmittel gewählt, so hat es sich als vorteilhaft erwiesen, wenn das molare Verhältnis der oben genannten Edukte und des Lösungsmittel zueinander etwa (0,8-1,2):(0,9-1,3):(14-22) beträgt. Als besonders wirksam hat sich ein Verhältnis von (0,9-1,1):(1-1,2):(16-19) erwiesen.

Als besonders vorteilhaft hat sich eine sehr langsame Zugabe des in einem Teil des verwendeten Lösungsmittels gelösten Diphenylacetylchlorids erwiesen, und zwar insbesondere unter beibehaltener niedriger Temperatur. Zur Vervollständigung der Reaktion wird noch einige Zeit bei gleicher Temperatur weitergerührt, jedoch möglichst nicht länger als etwa 4 Stunden. Als optimal hat sich eine Nachrührdauer von 1,5 bis 2,5 Stunden herausgestellt.

Zur Reinigung des auf diese Weise erhaltenen Rohprodukts wird aus einem geeigneten Lösungsmittel umkristallisiert. Hierbei ist es vorteilhaft, die Lösungsmittelmenge so zu wählen, daß das Produkt bereits in der Wärme auskristallisiert.

Bei Verwendung von Ethanol erreicht man dieses bei molaren Verhältnissen von etwa einem Mol Produkt bezogen auf 75 bis 125 Mol, insbesondere 85 bis 115 Mol, Lösungsmittel.

Je nach Wahl des Lösungsmittels, aus dem umkristallisiert wird, fällt das gereinigte Produkt bereits nach kurzem Abkühlen kristallin in der Modifikation vom Typ IV aus. Bei Verwendung von Ethanol ist dieses nach dem Abkühlen auf Temperaturen von etwa 55 bis 45 °C der Fall. Dabei hat es sich als vorteilhaft erwiesen, diese Temperatur während des gesamten Auskristallisierens beizubehalten.

Als besonders vorteilhaft hat sich bei der erfindungsgemäßen Verbindung erwiesen, daß sie aufgrund ihrer Struktur offenbar die Blut-Him-Schranke nicht passieren kann und daher kein Abhängigkeitspotential aufweist. Auch wurden bisher keine Nebenwirkungen gefunden, die die Nutzung der vorteilhaften Wirkungen für die beanspruchten Indikationen in irgendeiner Weise einschränken würden.

Die erfindungsgemäße Verbindung und ihre physiologisch unbedenklichen Salze können daher zur Herstellung pharmazeutischer Präparate verwendet werden, indem man sie zusammen mit mindestens einem Träger- oder Hilfsstoff und, falls erwünscht, mit einem oder mehreren weiteren Wirkstoffen in die geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z. B. orale oder rektale) oder parenterale Applikation eignen und mit der erfindungsgemäßen Verbindung nicht reagieren, beispielsweise Wasser, pflanzliche Öle, Benzylalkohole, Polyethylenglykole, Glycerintriacetat und andere Fettsäureglyceride, Gelatine, Sojalecithin, Kohlenhydrate wie Lactose oder Stärke, Magnesiumstearat, Talk oder Cellulose.

Zur oralen Anwendung dienen insbesondere Tabletten, Dragees, Kapseln, Sirupe, Säfte oder Tropfen. Von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wäßrige Lösungen, ferner Suspensionen, Emulsionen oder Implantate.

Der erfindungsgemäß beanspruchte Wirkstoff kann auch lyophilisiert und das erhaltene Lyophilisat z. B. zur Herstellung von Injektionspräparaten verwendet werden.

Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine, Diuretika, Antiphlogistika.

Die erfindungsgemäße Verbindung wird in der Regel in Analogie zu anderen bekannten, für die beanspruchten Indikationen im Handel ererhältlichen Präparaten verabreicht, vorzugsweise in Dosierungen zwischen etwa 1 mg und 50 mg, insbesondere zwischen 5 und 30 mg pro Dosierungseinheit. Die tägliche Dosierung liegt vorzugsweise zwischen etwa 0.02 und 20 mg/kg, insbesondere 0,2 und 0,4 mg/kg Körpergewicht.

Die spezielle Dosis für jeden einzelnen Patienten hängt jedoch von den verschiedensten Faktoren ab, beispielsweise vom Alter, Körpergewicht, allgemeinem Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Im folgenden werden Beispiele gegeben, die zur Veranschaulichung der Erfindung dienen.

Nachstehend sind alle Temperaturen in ° C angegeben.

### Vergleichsbeispiel

### N-Methyl-N-[(1S)-1-phenyl-2-((3S)-3-hydroxypyrrolidin-1-yl)-ethyl]-2,2-diphenylacetamid-hydrochlorid (Typ II)

In einer 500 ml- Apparatur werden 22 g 1-[(1S)-3-Hydroxypyrrolidin-1-yl]-(2S)-2-methylamino-2-phenyl-ethan vorgelegt und in 150 ml Tetrahydrofuran gelöst. Unter Rühren wird eine Lösung, bestehend aus 150 ml Tetrahydrofuran und 24,1 g Diphenylacetylchlorid, bei 10 - 20°C innerhalb von einer Stunde zu getropft, wobei zu Beginn sich ein Niederschlag bildet, der jedoch im Verlauf der Reaktion wieder in Lösung geht. Gegen Ende der Reaktion bildet sich erneut ein Niederschlag. Es wird weitere 12 Stunden bei Zimmertemperatur gerührt. Anschließend wird auf ca. 5°C gekühlt und das ausgefallene Produkt abgesaugt. Das abgetrennte Produkt wird mit etwa 100 ml Tetrahydrofuran nachgewaschen und getrocknet. Auf diese Weise werden 39 g Rohprodukt erhalten. Dieses wird mit etwa 250 ml Ethanol und 1 g Aktivkohle umkristallisiert.
Ausbeute: 33 g N-Methyl-N-[(1S)-1-phenyl-2-((3S)-3-hydroxypyrrolidin-1-yl)-ethyl]-2,2-diphenylacetamid-hydrochlorid (73,2 % der Theorie)
Schmelzpunkt: 196-200 °C
Schmelzwärme: 100 J/g
pKa: 7,4
Löslichkeit in Wasser bei 20 °C: 1,16 g /100 ml
Löslichkeit in Methanol bei 20 °C: 6,31 g /100 ml

### Beispiel 1

### N-Methyl-N-[(1S)-1-phenyl-2-((3S)-3-hydroxypyrrolldin-1-yl)-ethyl]-2,2-diphenylacetamid-hydrochlorid (Typ IV)

Zu einer auf 0 bis 8 °C abgekühlten Reaktionslösung, bestehend aus 9 g 1-[(1S)-3-Hydroxypyrrolidin-1-yl]-(2S)-2-methylamino-2-phenylethan und 40 ml Tetrahydrofuran, wird langsam innerhalb von 75 Minuten eine Lösung, bestehend aus 10,5 g Diphenylessigsäurechlorid und 17 ml Tetrahydrofuran, unter Rühren zugetropft. Anschließend wird noch 120 °C bei gleicher Temperatur nachgerührt. Das dabei als Niederschlag ausfallende Reaktionsprodukt wird abgesaugt und getrocknet. Auf diese Weise werden 17 g Rohprodukt erhalten, die aus 180 ml Ethanol umkristallisiert werden. Während dieses Umkristallisierens fällt das hergestellte Produkt bei 50 °C als stabiler Kristalltyp IV aus.
Ausbeute: 13 g N-Methyl-N-[(1S)-1-phenyl-2-((3S)-3-hydroxypyrrolidin-1-yl)-ethyl]-2,2-diphenylacetamid-hydrochlorid (70,6% der Theorie)
Schmelzpunkt: 220 - 225 °C
Schmelzwärme: 124 J/g
pKa: 7,4
Löslichkeit in Wasser bei 20 °C: 0,76 g /100 ml
Löslichkeit in Methanol bei 20 °C: 4,26 g /100 ml

Die nachfolgenden Beispiele betreffen pharmazeutische Zubereitungen:

### Beispiel A: Injektionsgläser

Eine Lösung von 100 g Wirkstoff und 5 g Dinatriumhydrogenphosphat werden in 3 I zweifach destilliertem Wasser mit 2 n Salzsäure auf pH 6.5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g Wirkstoff mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g Wirkstoff 9.38 g NaH₂PO₄·2H₂O, 28.48 g NA₂HPO₄·12H₂O und 0.1 g Benzalkoniumchlorid in 940 ml zweifach destilliertem Wasser. Man stellt auf pH 6.8 ein, füllt auf 1 I auf und sterilisiert durch Bestrahlung.

### Beispiel D: Salbe

Man mischt 500 mg Wirkstoff mit 99.5 g Vaseline unter aseptischen Bedingungen.

### Beispiel E: Tabletten

Ein Gemisch von 1 kg Wirkstoff, 4 kg Laktose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg Wirkstoff enthält.

### Beispiel H: Ampullen

Eine Lösung von 1 kg Wirkstoff in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. N-Methyl-N-[(1S-)-1-phenyl-2-((3S)-3-hydroxypyrrolidin1-yl-)-ethyl]-2,2-diphenyl-acetamid-hydröchlorid in der thermodynamisch stabilen Kristallmodifikation mit einem Schmelzpunkt von 220 - 225 °C.

2. Arzneimittel enthaltend eine Verbindung gemäß Anspruch 1

3. Verwendung des Arzneimittels gemäß Anspruch 2 zur Herstellung von Arzneimittelformulierungen mit der pharmakologischen Wirkung als kappa-Opiatagonist.

4. Verwendung des Arzneimittels gemäß Anspruch 2 zur Herstellung von Arzneimittelformulierungen zur Behandlung von entzündlichen Darmerkrankungen.

5. Verwendung des Arzneimittels gemäß Anspruch 2 zur Herstellung von Arzneimittelformulierungen zur Behandlung von Schmerzen und Schmerzüberempfindlichkeit auftretend bei Rückenleiden.

6. Verwendung des Arzneimittels gemäß Anspruch 2 zur Herstellung von Arzneimittelformulierungen zur Behandlung von Schmerzen, Schmerzüberempfindlichkeit und entzündlichen Reaktionen bei rheumatischen Erkrankungen, Brandverletzungen, Sonnenbrand oder Neurodermitis.

7. Verwendung des Arzneimittels gemäß Anspruch 2 zur Herstellung von Arzneimittelformulierungen zur Behandlung von postoperativen Schmerzen, Schmerzüberempfindlichkeit und eines postoperativen Ileus.

8. Pharmazeutische Zubereitung, **dadurch gekennzeichnet, daß** sie ein Arzneimittel gemäß Anspruch 2 enthält.

9. Verfahren zur Herstellung von N-Methyl-N-[(1S-)-1-phenyl-2-((3S)-3-hydroxypyrrolidin1-yl-)-ethyl]-2,2-diphenyl-acetamid-hydrochlorid nach Anspruch 1, **dadurch gekennzeichnet, daß**
a) 1-[(1S)-3-Hydroxypyrrolidin-1-yl]-(2S)-2-methylamino-2-phenylethan und Diphenylacetylchlorid bei niedrigen Temperaturen, insbesondere bei -5 bis 10 °C, miteinander umgesetzt werden, indem
b) in einem Lösungsmittel gelöstes Diphenylacetylchlorid langsam unter Beibehaltung der Temperatur zu dem in dem gleichen Lösungsmittel gelösten, in der Apparatur vorgelegten 1-[(1S)-3-Hydroxypyrrolidin-1-yl]-(2S)-2-methylamino-2-phenylethan zugegeben wird, und
c) im Anschluß an die Reaktion das erhaltene Rohprodukt aus einem Lösungsmittel in der Wärme umkristallisiert wird.

## Claims

1. N-Methyl-N-[(1S)-1-phenyl-2-((3S)-3-hydroxypyrrolidin-1-yl)ethyl]-2,2-diphenylacetamide hydrochloride in the thermodynamically stable crystal modification having a melting point of 220-225°C.

2. Medicament containing a compound according to Claim 1.

3. Use of the medicament according to Claim 2 for the production of pharmaceutical formulations with pharmacological action as a kappa-opiate agonist.

4. Use of the medicament according to Claim 2 for the production of pharmaceutical formulations for the treatment of inflammatory bowel disorders.

5. Use of the medicament according to Claim 2 for the production of pharmaceutical formulations for the treatment of pain and hypersensitivity to pain occurring in back conditions.

6. Use of the medicament according to Claim 2 for the production of pharmaceutical formulations for the treatment of pain, hypersensitivity to pain and inflammatory reactions in rheumatic disorders, burn injuries, sunburn or neurodermatitis.

7. Use of the medicament according to Claim 2 for the production of pharmaceutical formulations for the treatment of postoperative pain, hypersensitivity to pain and postoperative ileus.

8. Pharmaceutical preparation, **characterized in that** it contains a medicament according to Claim 2.

9. Process for the preparation of N-methyl-N-[(1S)-1-phenyl-2-((3S)-3-hydroxypyrrolidin-1-yl)ethyl]-2,2-diphenylacetamide hydrochloride according to Claim 1, **characterized in that**
a) 1-[(1S)-3-hydroxypyrrolidin-1-yl]-(2S)-2-methylamino-2-phenylethane and diphenylacetyl chloride are reacted with one another at low temperatures, in particular at -5 to 10°C, by
b) slowly adding diphenylacetyl chloride dissolved in a solvent to the 1-[(1S)-3-hydroxypyrrolidin-1-yl]-(2S)-2-methylamino-2-phenylethane dissolved in the same solvent and initially introduced into the apparatus while maintaining the temperature, and
c) following the reaction recrystallizing the crude product obtained from a hot solvent.

## Revendications

1. Chlorhydrate du N-méthyl-N-[(1S-)-1-phényl-2-((3S)-3-hydroxypyrrolidine-1-yl-)-éthyl]-2,2-diphénylacétamide sous sa forme cristalline thermodynamiquement stable, ayant un point de fusion de 220-225°C.

2. Médicament contenant un composé selon la revendication 1.

3. Utilisation du médicament selon la revendication 2 pour préparer des formulations médicamenteuses ayant un effet pharmacologique en tant qu'agoniste des κ-opiacés.

4. Utilisation du médicament selon la revendication 2 pour préparer des formulations médicamenteuses destinées au traitement des maladies inflammatoires de l'intestin.

5. Utilisation du médicament selon la revendication 2 pour préparer des formulations médicamenteuses destinées au traitement des douleurs et de l'hypersensibilité à la douleur apparaissant lors des maux de dos.

6. Utilisation du médicament selon la revendication 2 pour préparer des formulations médicamenteuses destinées au traitement des douleurs, de l'hypersensibilité à la douleur et des réactions inflammatoires en présence de maladies rhumatismales, de brûlures, de coups de soleil ou d'une névrodermite.

7. Utilisation du médicament selon la revendication 2 pour préparer des formulations médicamenteuses destinées au traitement des douleurs postopératoires, de l'hypersensibilité à la douleur et de l'iléus post-opératoire.

8. Préparation pharmaceutique, **caractérisée en ce qu'**elle contient un médicament selon la revendication 2.

9. Procédé de préparation du chlorhydrate du N-méthyl-N-[(1S-)-1-phényl-2-((3S)-3-hydroxypyrrolidine-1-yl-)-éthyl]-2,2-diphénylacétamide selon la revendication 1, **caractérisé en ce que**.
a) on fait réagir l'un avec l'autre à de basses températures, en particulier de -5 à 10°C, du 1-[(1S)-3-hydroxypyrrolidine-1-yl]-(2S)-2-méthylamino-2-phényléthane et du chlorure de diphénylacétyle, ce pour quoi
b) tout en conservant la température, on ajoute lentement du chlorure de diphénylacétyle dissous dans un solvant, au 1-[(1S)-3-hydroxy-pyrrolidine-1-yl]-(2S)-2-méthylamino-2-phényléthane, dissous dans le même solvant et déjà en place dans l'appareillage, et
c) après la réaction, on recristallise à chaud dans un solvant le produit brut obtenu.
